# EUROPEAN PATENT APPLICATION

(11) **EP 1 182 263 A1**
(43) Date of publication of application: **27.02.2002**
(21) Application number: 00117457.2
(22) Date of filing: 11.08.2000
(51) Int. Cl.: C12Q 1/48, G01N 33/68

(54) **Process for detecting threonine/serine kinase activity**

(71) Applicant: Evotec OAI AG, 22525 Hamburg (DE); Pfizer Limited, Sandwich, Kent CT13 9NJ (GB)
(72) Inventor: Krämer, Joachim, c/o EVOTEC BioSysteme AG, 22525 Hamburg (DE); Bethell, Richard, Sandwich, Kent CT13 9NJ (GB); Mander, Thomas, c/o EVOTEC BioSystems AG, 22525 Hamburg (DE); Benson, Neil, Sandwich, Kent CT13 9NJ (GB); Boyd, Helen, Sandwich, Kent CT13 9NJ (GB); Greengrass, Pam, Sandwich, Kent CT13 9NJ (GB); Kinlock, Ross, Sandwich, Kent CT13 9NJ (GB)
(74) Representative: Draudt, Jutta, Dr.

(57) **Abstract**

The present invention describes a process for detecting threonine or serine kinase activity in an immunoassay using a pre-phosphorylated substrate.

## Description

The present invention relates to a process for detecting threonine or serine (threonine/serine) kinase activity in an immunoassay. The invention further relates to a kit for carrying out the assay and to a luminescently labelled ligand.

Protein kinases catalyse the transfer of the γ-phosphate group from ATP to a serine, threonine or tyrosine residue of an acceptor protein. These enzymes play an important role in signal transduction within cells. Detecting the activity of protein kinases would be useful for the high-throughput screening of chemical libraries. Inhibitors or activators of kinase activity, particularly low-molecular weight compounds, could eventually be developed into drugs used for the treatment of e.g. ischaemic heart disease, liver failure, diabetic neuropathies, stroke, neurodegenerative disorders including Parkinsons disease and Alzheimers disease, inflammatory diseases including asthma, rheumatoid arthritis, inflammatory bowel disease, septic shock and cancer.

It is known that mitogen-activated protein kinases (MAPK) (also referred to as stress activated protein kinases, SAPK) mediate many of the cellular effects of growth factors, cytokines and stress, leading to cell growth, differentiation and oncogenesis. MAPK/SAPK activation requires dual phosphorylation on threonine and tyrosine within the motif threonine-Xaa-tyrosine, where Xaa represents proline in the c-Jun NH2-terminal kinases (JNKs). This activation is catalysed by several MAPK-kinases (e.g. MKK4(SKK1) or MKK7 (SKK4)) which phosphorylate both the threonine and tyrosine residues of the threonine(183)-proline-tyrosine(185) motif within the active site loop of JNK1/2/3. The MKK7 kinase has a high preference for the threonine residue whereas MKK4 preferentially phosphorylates tyrosine. This synergistic activation of JNK1/2/3 is described by Lawler S. et al in *Current Biology* 8, 1387 to 1390 (1998) and Fleming Y et al. submitted to Biochemical Journal, 2000. Up to now the identification of an appropriate substrate which could be used to screen for specific MKK7 inhibitors is complicated by the fact that there are no synthetic substrate peptides described in the literature. The situation becomes even more critical in homogeneous assays as a potential MKK7 substrate peptide should have both a reasonable Km for MKK7 and a high affinity for the phospho-threonine specific antibody in parallel. Although generic anti-phospho-tyrosine antibodies with high affinity and specificity are available for tyrosine-directed kinases (e.g. p60c-src kinase), attempts to develop generic anti-phosphothreonine/anti-phospho-serine antibodies for the same purpose have been to date less successful.

Antibodies which bind to phosphorylated threonine or serine residues with high affinity only recognize the phosphorylated amino acid in the context of the surrounding peptide sequence. For antibodies binding to JNK1/2/3 or peptide derivatives thereof, recognition is therefore dependent on two criteria: 1) MKK7-dependent phosphorylation of threonine and 2) phosphorylation of the tyrosine amino acid residue. If one of the two criteria is not fulfilled, an antibody raised against fully activated JNK1/2/3 will not specifically detect the phosphorylation site.

In *Anal. Biochem*. 255, 257 to 262 (1998) a fluorescence polarization (FP) competition immunoassay for tyrosine kinases using an appropriate substrate for this kinase is described. The kinase reaction was performed by incubation of the peptide substrate with ATP and lymphoid T-cell protein tyrosine kinase followed by termination of the reaction with EDTA plus a fluorescein-phosphopeptide. Following the addition of an anti-phosphotyrosine antibody, the fluorescence polarization signal was measured. The phosphorylated product formed in the assay competes with the fluorescein-phosphopeptide for binding to the anti-phosphotyrosine antibody. The kinase activity results in a reduction of the FP signal and the FP signal is therefore inversely proportional to the phosphorylated product formed in the reaction.

However, such an approach for a generic assay principle for serine/threonine kinases is not realizable to date. This is because there are no anti-phospho-serine/-threonine antibodies available that bind specifically, and with high affinity, to phosphoserine or phosphothreonine residues in the absence of additional specific amino acid sequences adjacent to the phosphorylated amino acid.

It was therefore the object of the present invention to establish an assay method for detecting serine/threonine kinases. The assay method is highly reliable and simple to perform without the need to develop specific high affinity anti-phospho-serine/-threonine antibodies.

This object has been solved by the assay process according to the features of claim 1.

The present invention relates to a process for detecting threonine or serine kinase activity in an immunoassay which comprises the following steps:
a) providing a protein or peptide comprising the sequence motif

   - Z - X - Y - or - Y - X - Z -

   wherein
   Z = threonine or serine
   X = a sequence of between 1 and 1000 amino acids which may be the same or different
   Y = tyrosine, threonine or serine
   as a substrate for threonine or serine kinases, said protein or peptide being pre-phosphorylated at the Y position;
b) incubating the peptide or protein with a phosphate donor and a threonine or serine kinase to form a bis-phosphorylated protein or peptide;
c) adding an antibody having a specificity to the peptide/protein that has been phosphorylated at threonine or serine in the Z position; and
d) detecting the threonine or serine kinase activity.

Particularly, the antibody has a specificity to the bis-phosphorylated product of the kinase reaction. By detecting the presence, absence or amount of the product-antibody complex, a threonine or serine kinase activity can be measured.

The subclaims define preferred embodiments of the process of the present invention.

The present invention also relates to a kit for detecting threonine or serine kinase activity in an immunoassay which comprises the following components:
- a threonine or serine kinase;
- a mono-phosphorylated substrate as defined above;
- an antibody as defined above; and
- reaction buffers including a phosphate donor.

The process of the present invention as well as the kit may be used for screening for specific modulators of serine or threonine kinase activity. It is particularly suitable for screening compound libraries in order to locate molecules which inhibit or activate kinases. These molecules may be promising candidates for designing drugs used for the treatment of e.g. ischaemic heart disease, liver failure, diabetic neuropathies, stroke, neurodegenerative disorders including Parkinsons disease and Alzheimers disease, inflammatory diseases including asthma, rheumatoid arthritis, inflammatory bowel disease, septic shock and cancer.

The accompanying figures illustrate the present invention. Abbreviations are used as follows: P1° H-Lys-Phe-Met-Met-Thr-Pro-pTyr-Val-Val-Thr-Arg-NH2 P1*          H-Lys-Phe-Met-Met-pThr-Pro-Tyr-Val-Val-Thr-Arg-NH2 P1*°          H-Lys-Phe-Met-Met-pThr-Pro-pTyr-Val-Val-Thr-Arg-NH2 
TAMRA-P1*° 5-TAMRA-AEEA-Lys-Phe-Met-Met-pThr-Pro-pTyr-Val-Val-Thr-Arg-
NH2
 TAMRA 5'-(6-carboxytetramethylrhodamine)
AEEA 8-amino-3,6-dioxaoctanoic acid linker
In the figures the following is shown:
- Figure 1: is a schematic drawing of a preferred embodiment of the principle of the assay of the present invention.
- Figure 2: is a schematic diagram showing the binding of the polyclonal JNK antibody to TAMRA-labelled P1°*-peptide; as a control, no binding is measured for TAMRA-labelled P1°-peptide.
- Figure 3: is a schematic diagram showing the competition of the binding of the polyclonal JNK antibody to the TAMRA-labelled P1°*-peptide using mono-phosphorylated P1°-and P1*-peptides and double-phosphorylated P1°*-peptide (determination of the IC50 for the P1°*-peptide competitor).
- Figure 4: is a schematic diagram showing the competition of the binding of the polyclonal JNK antibody to the TAMRA-labelled P1°*-peptide in presence of 100 µM P1°-peptide (substrate) using P1°*-peptide (determination of the IC50 for the P1°*-peptide competitor under MKK7 kinase assay conditions).
- Figure 5: is a schematic diagram showing the phosphorylation of P1°-peptide substrate by MKK7 kinase at different P1°-peptide concentrations (determination of the Km for P1°-peptide).
- Figure 6: is a schematic diagram showing the phosphorylation of P1°-peptide substrate by MKK7 kinase at different ATP concentrations (determination of the Km for ATP).
- Figure 7: is a schematic diagram showing the inhibition of the MKK7 kinase-dependent phosphorylation of P1°-peptide substrate by Staurosporine (determination of the IC50 for Staurosporine).

According to the invention, a protein or peptide comprising the sequence motif -Z-X-Y- or -Y-X-Z-, wherein Z = threonine or serine; X = a sequence of between 1 and 1000 amino acids which may be the same or different; Y = tyrosine, threonine or serine, is used as a substrate for the threonine or serine kinase. According to the present invention said protein or peptide is pre-phosphorylated at the Y position. It has been shown that the proteins and peptides being pre-phosphorylated at the Y residue can be successfully used as synthetic substrates for threonine or serine kinases.

In the sequence motif, any amino acid or sequence of amino acids (X) can be inserted between Z and Y. The number of amino acids is preferably in the range of 1 to 1000 amino acids. A range of 1 to 1000 is preferred to include both linear and conformational antibody epitopes. X is particularly at least one amino acid, any other short amino acid sequences having at least two amino acids, such as oligopeptides, being also included. Preferably, X is proline or glutamate or glycine.

The antibody used is usually a monoclonal or polyclonal antibody.

It has been shown that a particularly preferred antibody is a polyclonal antibody specific for activated JNK. Such antibodies are commercially available.

It has been revealed that the antibody specifically recognizes a phosphorylated threonine or serine residue at the Z position within both a synthetic substrate peptide or activated JNK.

The immunoassay of the present invention may be performed as a direct binding immunoassay, preferably a homogeneous direct binding immunoassay.

In the direct binding immunoassay, a labelled peptide or protein or a labelled antibody is used. The labelling may be carried out according to conventional standard techniques. Preferably, the peptide/protein or antibody is labelled using a luminescent or a radioactive tag or by using specific labelling molecules such as a reporter enzyme or an affinity ligand.

The assay of the present invention may be also be performed as a competition immunoassay, preferably a homogeneous indirect binding immunoassay.

In this indirect binding immunoassay, a labelled double-phosphorylated ligand is added to compete with the double-phosphorylated peptide or protein for binding to the antibody. The ligand is preferably labelled using a luminescent or a radioactive tag or by using specific labelling molecules such as a reporter enzyme or an affinity ligand.

In case of using a protein containing the above motif, the JNK protein is preferably used, which is the c-Jun N-terminal kinase, and which is also known in the literature as the stress-activated protein kinase 1 (SAPK1).

When it is more convenient to use a peptide substrate, the peptide sequence is preferably selected from the active-site loop, e.g. for MKK7 from the JNK1/2/3 active site. For instance, said peptide for MKK7 comprises or is composed of the amino acid sequence H-Lys-Phe-Met-Met-Thr-Pro-pTyr-Val-Val-Thr-Arg-NH₂, wherein p means phosphorylated.

When the incubation of the protein or peptide is carried out in the presence of a threonine kinase, the threonine kinase is preferably a mitogen-activated protein kinase kinase(MKK), alternatively called stress activated protein kinase (SKK). More particularly, the kinase is MKK7/SKK4.

According to one embodiment of the process of the present invention, an antibody is added preferably at the start of the reaction having a specificity to the phosphorylated threonine or serine, preferably to the bis-phosphorylated sequence. During the enzyme reaction any double phosphorylated product formed is bound by the antibody. Then, at the end of the enzyme reaction, a labelled, double phosphorylated ligand is added, either with or without the concomitant addition of a reagent to stop the reaction, to compete with the double phosphorylated protein or peptide for binding to the antibody. This indirect assay principle is based on the fact that if the substrate becomes double phosphorylated by the kinase such as MKK7, it will compete with the labelled, double phosphorylated peptide-antibody complex. This strategy has the advantage of using non-labelled substrates and a fixed antigen-antibody complex that gives the read-out.

Usually, the double phosphorylated ligand is labelled by common techniques, using a luminescent or a radioactive tag or by molecules such as a reporter enzyme or an affinity ligand. Preferably, the ligand is labelled by a fluorescent dye. Particularly, the ligand comprises or is composed of the sequence 5-TAMRA-AEEA-Lys-Phe-Met-Met-pThr-Pro-pTyr-Val-Val-Thr-Arg-NH₂.

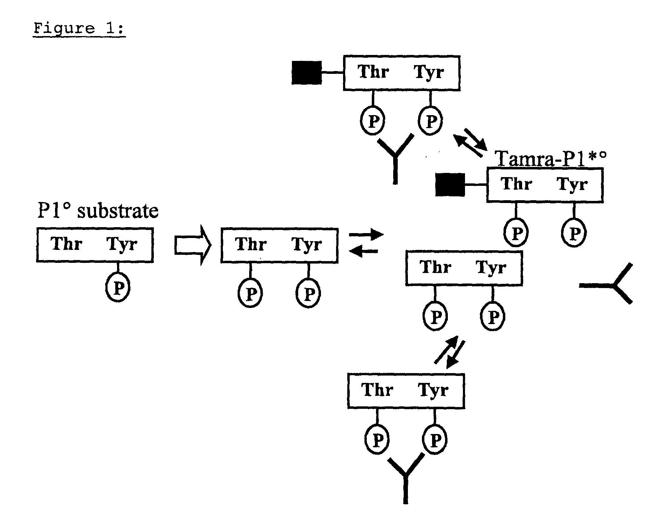

In Fig. 1 the indirect assay principle is illustratively shown. The P1° substrate having the amino acid sequence H-Lys-Phe-Met-Met-Thr-Pro-pTyr-Val-Val-Thr-Arg-NH₂ (p means phosporylated) becomes phosphorylated at the threonine residue in the presence of ATP and the MKK7 kinase. This double-phosphorylated peptide (ligand) competes with the labelled TAMRA-P1*° peptide having the sequence 5-TAMRA-AEEA-Lys-Phe-Met-Met-pThr-Pro-pTyr-Val-Val-Thr-Arg-NH₂ for binding to the antibody.

In a preferred embodiment, the assay may be conveniently performed as a fluorescence polarization immunoassay.

According to the invention a kit for detecting threonine or serine kinase activity is provided. The kit comprises preferably the following components:
1. a threonine or serine kinase such as natural or recombinant enzyme;
2. a substrate in form of a monophosphorylated-peptide or mono-phosphorylated native protein;
3. an anti-phospho-threonine/-serine antibody;
4. reaction buffers including either MnCl₂ or MgCl₂, and ATP;
5. reaction vials; and
6. protocol

The kit preferably further comprises a luminescently labelled ligand, which ligand comprises a sequence motif

-Z-X-Y- or -Y-X-Z-

wherein
Z = threonine or serine
X = a sequence of between 1 and 1000 amino acids which may be the same or different
Y = tyrosine, threonine or serine
as a competitor, said competitor being pre-phosphorylated at the Z and Y positions.

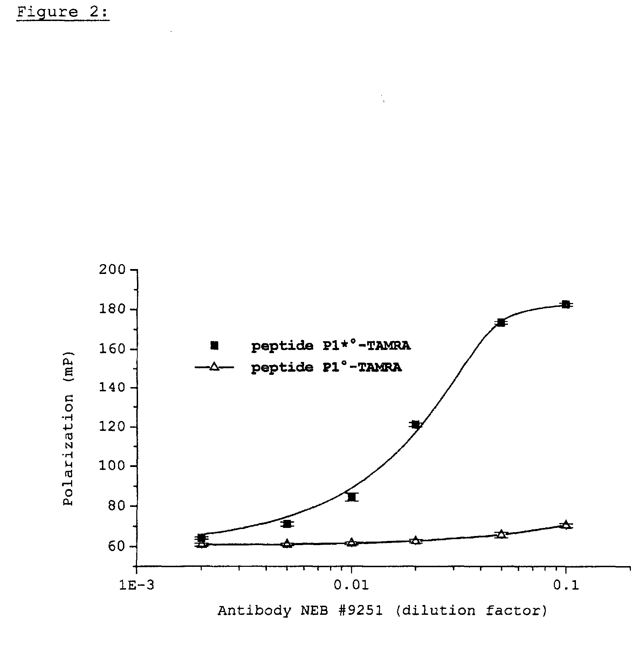

It has been revealed that the commercially available polyclonal anti-phospho-kinase antibodies are particularly useful in the assay of the present invention. As can be seen in Fig. 2 (ref. Example 1), the polyclonal phospho-JNK antibody detects all three isoforms of the JNK proteins only when activated by dual phosphorylation at threonine₁₈₃ and tyrosine₁₈₅. The polarization values dramatically increase when using the double-phosphorylated peptide substrate P1*° in contrast to mono-phosphorylated labelled peptide P1° (sequence see above).

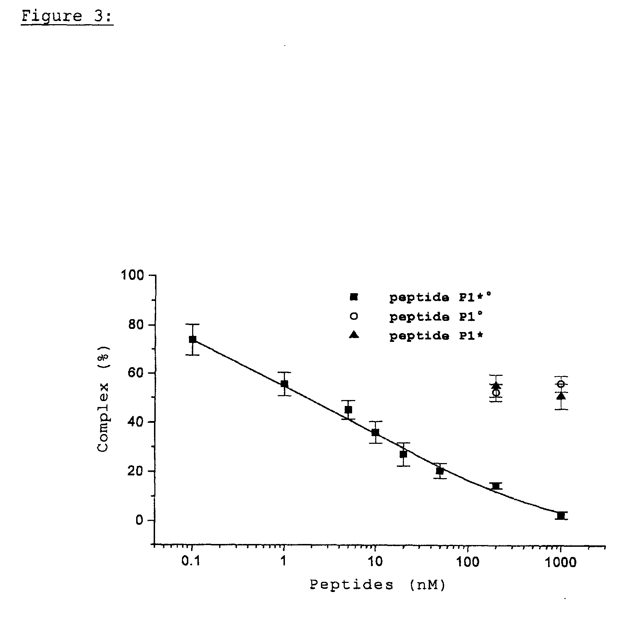

In Fig. 3 (ref. Example 2) the binding of the same polyclonal antibody to TAMRA-labelled peptide P1*°(5 nM) in competition with the peptides P1° (mono-phosphorylated, sequence see above), peptide P1* (mono-phosphorylated having the sequence H-Lys-Phe-Met-Met-pThr-Pro-Tyr-Val-Val-Thr-Arg-NH₂) or peptide P1*° (double phosphorylated, sequence see above) is presented. As can be deduced from the complex formation, only bis-phosphorylated peptide P1*⁰ is able to compete effectively with the ligand for binding to the antibody while the other mono-phosphorylated peptides displace less than 50% of the bound ligand even at high concentrations (up to 1 µM).

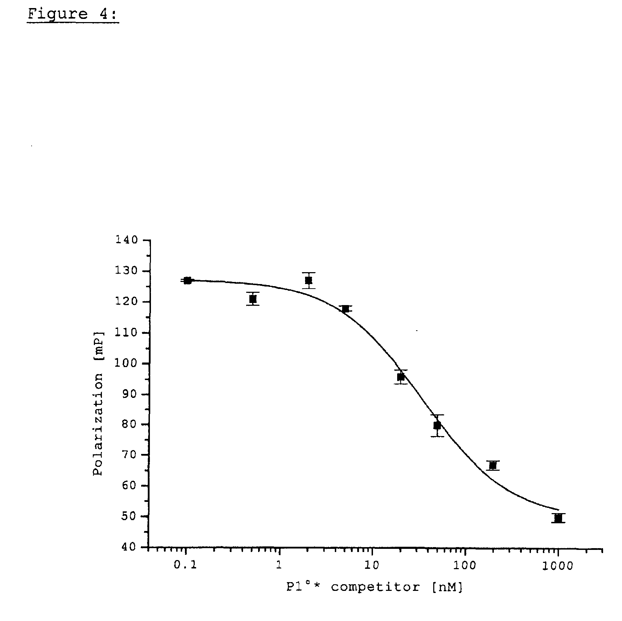

In Fig. 4 (ref. Example 3) the binding of the same polyclonal antibody to TAMRA-labelled peptide P1*°(5 nM) is displaced by bis-phosphorylated competitor peptide in presence of P1° peptide substrate (100 µM). These conditions reflect the concentration at which P1° peptide is used as a substrate for the MKK7 kinase. As can be seen from the result, the presence of mono-phosphorylated P1° peptide does not reduce the dynamic range of the described kinase assay.

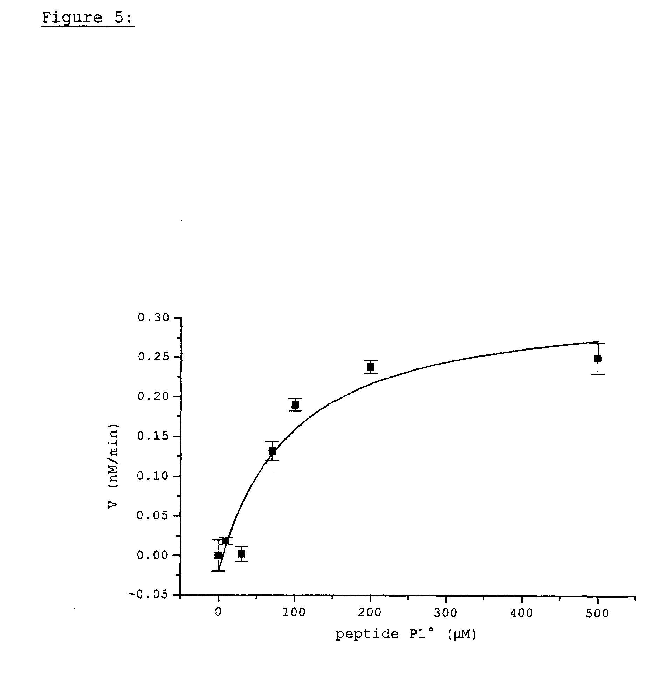

In Fig. 5 (ref. Example 4) the rate of phosphorylation of P1° peptide substrate by MKK7 kinase is determined at various P1° peptide concentrations. Efficient phosphorylation of P1° peptide is measured. The formed double-phosphorylated P1*° peptide product can be detected as it competes with the TAMRA-labelled P1*° peptide for the binding to the same polyclonal phospho-JNK antibody. As a results, a drop of the fluorescence polarization is detected which is inversely proportional to MKK7 kinase activity. From the detected signals, the Michaelis-Menten constant (Km), reflecting the half-maximal phosphorylation rate, was determined for P1° peptide.

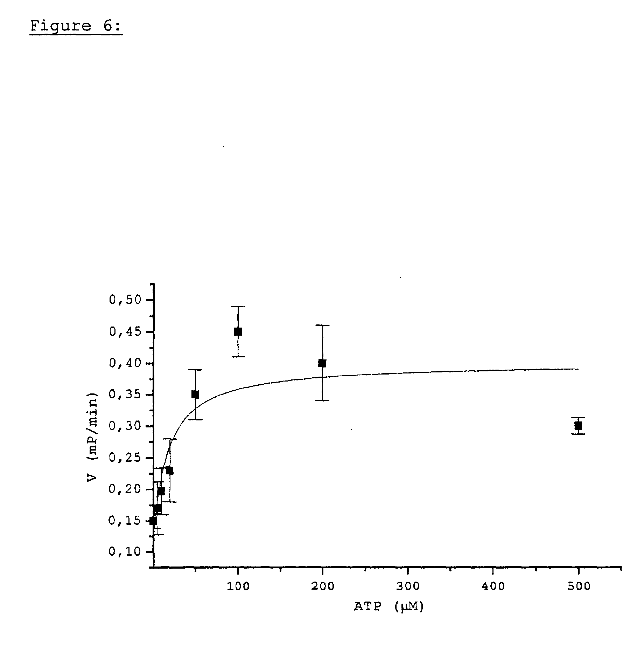

In Fig. 6 (ref. Example 5) the rate of phosphorylation of P1° peptide substrate by MKK7 kinase is determined at different ATP concentrations. Efficient phosphorylation of P1° peptide is dependent on the optimal ATP concentration. The formation of the bis-phosphorylated P1*° peptide product is detected using the same competition assay as described in Fig 5. From the detected fluorescence polarization signals, the Michaelis-Menten constant (Km), reflecting the half-maximal phosphorylation rate, was determined for ATP.

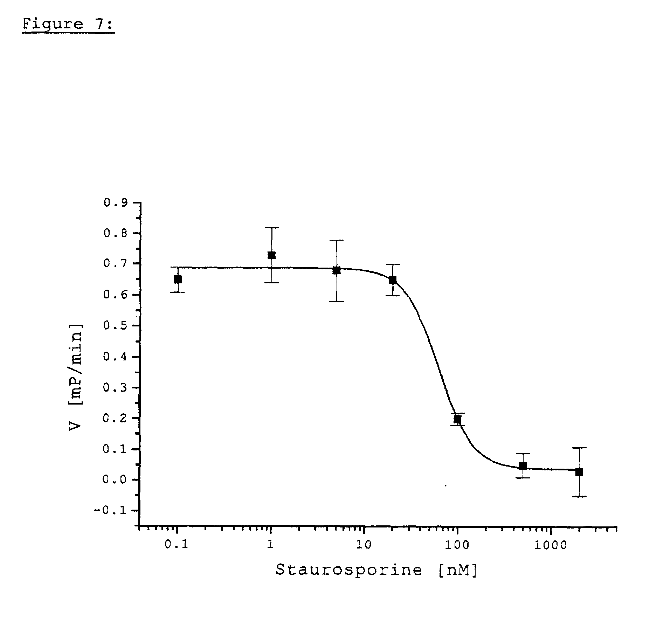

In Fig. 7 (ref. Example 6) the inhibition of the phosphorylation of P1° peptide substrate by MKK7 kinase is determined for the kinase inhibitor staurosporine. The inhibition of MKK7 kinase is detected using the same competition assay as described in Fig 5. As a result, no competition of the TAMRA-labelled P1*° peptide from the same polyclonal phospho-JNK antibody is detected at high Staurosporine concentrations. The half-maximal inhibitory concentration (IC50) was calculated for Staurosporine.

The following examples illustrate the assay of the present invention.

### Example 1:

### Measurement of the binding affinity (KD) of antibody NEB #9251:

This polyclonal antibody detects all three isoforms of the SAPK1/JNK proteins only when they are activated by dual phosphorylation at threonine₁₈₃/tyrosine₁₈₅. Binding of polyclonal phospho-JNK antibody (at a dilution of 1:20) from NEB to TAMRA-labelled P1°- and P1*° peptides (each 5 nM) was measured by fluorescence polarization.

Affinity of polyclonal phospho-JNK antibody for peptide P1*°-TAMRA: K_{D} = 2.6 nM

The results of this experiment are illustrated in Fig.2

### Example 2:

### Determination of IC50 for Peptide P1°:

Competition of NEB ab-P1°*-TAMRA complex with P1°* peptide was measured by fluorescence correlation spectroscopy: Binding of polyclonal phospho-SAPK1/JNK antibody from NEB (1:20 diluted) to TAMRA-labelled peptide P1*° (5 nM) was competed with non-fluorescent, double-phosphorylated peptide P1*°. Control peptides: mono-phosphorylated P1° and P1*.
Peptide P1*°: IC₅₀ = 5.0 nM ± 3.3 nM

The results of this experiment are illustrated in Fig.3

### Example 3:

### Determination of IC50 for Peptide P1°(under MKK7 enzyme assay conditions).

Competition of NEB ab: : P1°*-TAMRA complex with P1°* in presence of P1° substrate peptide (reflecting MKK7 assay conditions) was measured by fluorescence polarization: Binding of polyclonal phospho-JNK antibody from NEB #9251 (at a dilution of 1:20) to TAMRA-labelled peptide P1*° (5 nM), competition with P1°* peptide (0.1, 0.5, 2, 5, 20, 50, 200, 2000 nM) in Hepes buffer, MgCl₂ 10 mM, ATP 20 µM, Pluronic 0.1%, P1° substrate peptide 100 µM.
Peptide P1*°: IC₅₀ = 35.1 nM ± 10.6 nM

The results of this experiment are illustrated in Fig.4

### Example 4:

### Determination of Km for peptide P1°(Read-out: fluorescence polarization):

Assay conditions: MKK7 kinase (80 nM), P1° peptide (0, 10, 30, 70, 100, 200, 500 µM), ATP (100 µM), NEB antibody #9251 (1:20) diluted and P1°* peptide-TAMRA (5 nM) at RT.
Kₘ = 94 nM ± 59 nM, Vₘₐₓ = 94.6 nM/min ± 0.03 nM/min

The results of this experiment are illustrated in Fig.5

### Example 5:

### Determination of Km for ATP (Read-out: fluorescence polarization):

Assay conditions: MKK7 (80 nM), P1° peptide (100 µM), ATP (0, 1, 5, 10, 20, 50, 100, 200, 500 µM), incubation time to 2h at RT, polyclonal active-JNK antibody from NEB #9251 (1:20 dilution) and P1°* peptide-TAMRA at (5 nM).
Kₘ = 17.3 nM ± 18.1 nM, Vₘₐₓ = 0.28 mP/min ± 0.07 mP/min

The results of this experiment are illustrated in Fig.6

### Example 6:

### Inhibition of MKK7 activity with Staurosporine (Read-out: fluorescence polarization):

Assay conditions: MKK7 (0.3µM), P1° peptide (100 µM), ATP (10 µM), Staurosporine (0, 0.05, 0.2, 1, 2, 5, 10 µM), polyclonal active-JNK antibody from NEB #9251 (1:20 dilution) and P1°* peptide-TAMRA at (5 nM).
IC₅₀ = 63.3 nM ± 10.2 nM

The results of this experiment are illustrated in Fig.7

### Example 7:

### Fluorescence Polarization measurements of MKK7 activity (time course):

In this experiment, the time course has been investigated at P1° substrate concentrations of 100 µM. The reactions were stopped with EDTA.

The following reagents have been used (the compositions of the buffers and specifications of the reagents are explained in more detail later on):
Hepes buffer, MgCl₂ 10 mM, ATP 20 µM, P1° peptide at 100 µM, NEB antibody # 9251 1:20 dilution, P1°*-TAMRA 5 nM, Pluronic 0.1%, MKK7 at 0, 0.005, 0.02, 0.08, 0.2, 0.5 and 2 µM.
After addition of MKK7, the enzyme reactions were performed in Nunc chambers at RT. The samples were continuously measured at different time points by Fluorescence polarization.

| | **0 h** |
|---|---|
| **Conjugate: P1°*-Tamra** | 39.7 |
| **High: P1°*-Tamra + NEB ab (1:20)** | 139.1 |
| **Low: P1°*-Tamra + NEB ab (1:20) + P1°* 200 nM** | 84.5 |

| ***100 µM P1°*** | **0 h** | **0.5 h** | **1 h** | **1.5 h** | **3 h** |
|---|---|---|---|---|---|
| **MKK7: 0.0 µM** | 134.7 | 139.1 | 139.7 | 140.2 | 144.8 |
| **MKK7: 0.005 µM** | 131.5 | 137.4 | 136.8 | 136.9 | 138.1 |
| **MKK7: 0.02 µM** | 135.4 | 140.3 | 140.9 | 139.7 | 140.0 |
| **MKK7: 0.08 µM** | 133.5 | 137.6 | 135.8 | 128.4 | 115.2 |
| **MKK7: 0.2 µM** | 135.3 | 132.1 | 121.9 | 113.8 | 104.7 |
| **MKK7: 0.5 µM** | 135.3 | 125.5 | 115.3 | 108.8 | 106.6 |
| **MKK7: 1.0 µM** | 135.7 | 119.6 | 112.2 | 105.3 | 108.4 |
| FP values are given in mP. | | | | | |

### Example 8:

### Fluorescence Polarization measurements of MKK7 activity (time course):

In this experiment the assay conditions have been optimized. The reactions were stopped with EDTA.

Two different assay strategies were investigated: first, the NEB read-out antibody was included in the enzyme reaction (STOP solution: contains only EDTA and P1°*-TAMRA); second, the NEB read-out antibody was included in the STOP solution.

The following reagents have been used (explanations later on): Hepes buffer, MgCl₂ (10 mM), ATP (20 µM), P1° peptide (100 µM), polyclonal antibody NEB #9251 (1:20), P1°*-TAMRA (5 nM), Pluronic (0.1%), BSA (0.01%), MKK7 at (0.04, 0.08 and 0.2 µM), EDTA (10 mM) (all final conc.).

The enzyme reactions were performed in Eppendorf reaction tubes at RT. Aliquots of 20 µl were withdrawn from the reactions at different time points and mixed with 10 µl STOP solution. The stopped reactions were incubated at RT for at least 30 min and measured by Fluorescence Polarization.

Results:

| | **0 h** |
|---|---|
| **Conjugate: P1°*-Tamra** | 33.2 |
| **High: P1°*-Tamra + NEB ab (1:20)** | 145.7 |
| **Low: P1°*-Tamra + NEB ab (1:20) + P1°* 200 nM** | 71.5 |

| ***NEB ab in reaction*** | **0 h** | **0.5 h** | **1 h** | **1.5 h** | **2 h** | **3 h** |
|---|---|---|---|---|---|---|
| **MKK7: 0.04 µM** | 150.0 | 128.5 | 125.7 | 112.9 | 109.7 | 78.6 |
| **MKK7: 0.08 µM** | 138.5 | 122.5 | 105.9 | 86.4 | 84.5 | 65.0 |
| **MKK7: 0.2 µM *NEB ab in STOP sol.*** | 137.2 | 101.4 | 83.1 | 78.1 | 72.1 | 59.4 |
| **MKK7: 0.04 µM** | 147.2 | 141.5 | 140.2 | 135.8 | 134.2 | 138.2 |
| **MKK7: 0.08 µM** | 143.3 | 139.1 | 133.6 | 122.1 | 115.8 | 125.0 |
| **MKK7: 0.2 µM** | 148.2 | 130.2 | 114.0 | 102.8 | 99.2 | 119.4 |
| FP values are given in mP. | | | | | | |

### Example 9:

### Fluorescence Polarization measurements of MKK7 activity (time course) in Nanocarrier plates (1.2 µl volume):

### Assay conditions (end conc.):

1. P1° substrate 100 µM, polyclonal antibody NEB #9251 (1:20), ATP 20 µM, MgCl 10 mM
2. MKK7 enzyme: 0, 25, 50, 75, 100, 200, 350, 500 nM
3. STOP solution: EDTA 10 mM, P1°*-TAMRA (5 nM)

### Stock solutions:

1. P1° substrate 125 µM, NEB ab (1:13.3), ATP 25 µM, MgCl 12.5 mM
2. MKK7 enzyme: 2500 nM
3. STOP solution: EDTA 60 mM, P1-TAMRA 30 nM

### Solutions 1. - 3. in Hepes buffer, Pluronic 0.1%, BSA 0.01%.

It has been revealed that bis-phosphorylated-TAMAR-P1*°-peptide (ligand) is bound by the polyclonal anti-active JNK-antibody, which is called "high" or "positive control".

Bis-phosphorylated peptide P1°* can be used as competitor, resulting in free labelled ligand called "low" or "negative control". This can be seen in the following table 1.

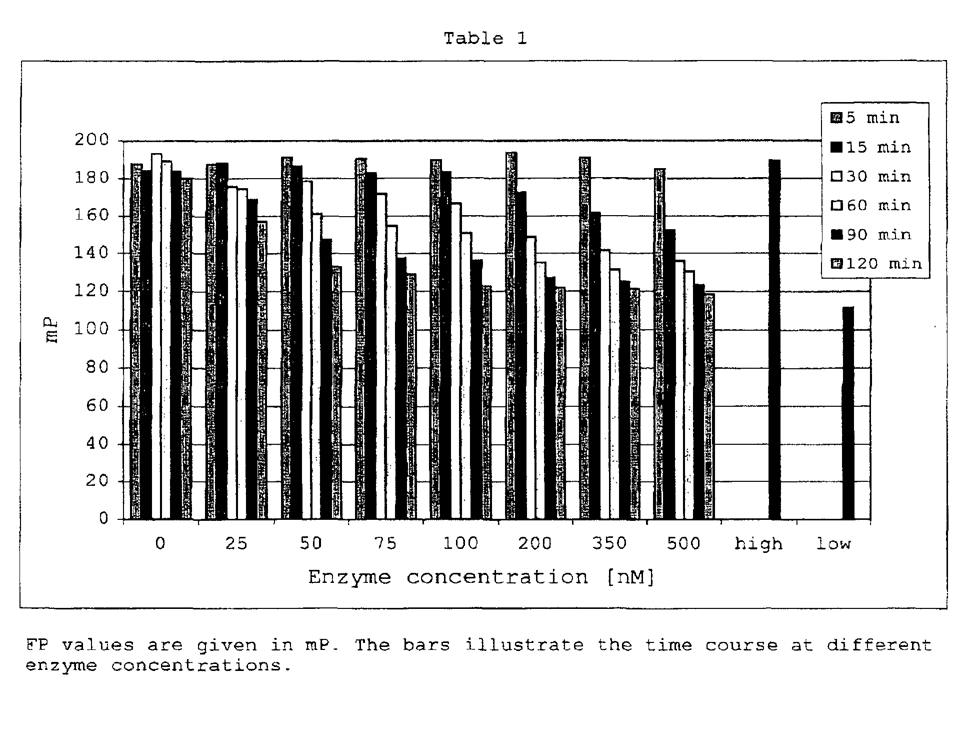

### List of reagents of the MKK7 kinase assay:

**List of used JNK1-peptides:**

P1°          H-Lys-Phe-Met-Met-Thr-Pro-pTyr-Val-Val-Thr-Arg-NH2

P1*          H-Lys-Phe-Met-Met-pThr-Pro-Tyr-Val-Val-Thr-Arg-NH2

P1*°          H-Lys-Phe-Met-Met-pThr-Pro-pTyr-Val-Val-Thr-Arg-NH2

TAMRA-P1*° 5-TAMRA-AEEA-Lys-Phe-Met-Met-pThr-Pro-pTyr-Val-Val-Thr-Arg-
NH2

### Ligand:

TAMRA-P1*°-Peptide: 5-TAMRA-AEEA-Lys-Phe-Met-Met-pThr-Pro-pTyr-Val-Val-Thr-Arg-NH2
Supplier: Give synthesis method and instrument name and
manufacturer EVOTEC inhouse solid-phase peptide synthesis HK-03-65-P1-13; M = 2089 g/mol; MALDI (2092.18)
1mM stock solution in 100% DMSO
The stock should be aliquoted in 10 µl aliquots and kept at - 20°C.
5nM working solution.

### Competitor:

P1*°-Peptide: H-Lys-Phe-Met-Met-pThr-Pro-pTyr-Val-Val-Thr-Arg-NH2
Supplier: EVOTEC inhouse solid-phase peptide synthesis
HK-03-60-P1-7; M = 1532 g/mol; MALDI (1531.88)
10mM stock solution in 100% DMSO
The stock should be aliquoted in 10 µl aliquots and kept at - 20°C.
200µM working solution.

### Substrate: MKK7 kinase substrate

P1°-Peptide: H-Lys-Phe-Met-Met-Thr-Pro-pTyr-Val-Val-Thr-Arg-NH2
Supplier: EVOTEC in house solid-phase peptide synthesis
HK-03-58-HF; M = 1451 g/mol; MALDI (1453.57)
10mM stock solution in 100% DMSO
The stock should be aliquoted in 10 µl aliquots and kept at - 20°C.
100µM working solution.

### Enzyme:

MKK7 kinase Supplier: Upstate Biotech cat. no. 14-366, lot no. 19105 and no. 20658.
See enzyme activity specifications on the respective certificate of analysis.
Specific activity: Approximately 20 Units/mg when max. activated.
20 µg of enzyme (9 µM) in 50µl of 50 mM Tris-HCl, pH 7.5, 150 mM NaCl, 270 mM sucrose, 0.1 mM EGTA, 0.1% 2-mercaptoethanol, 0.03% Brij-35, 1 mM benzamidine, 0.2 mM PMSF.
The stock should must be kept at -70°C where it will be stable for 6 months. Freezing and thawing cycles must be avoided.

### Anti active SAPK1/JNK-specific antibody:

### New England Biolabs, U.S.A.: NEB #9251

Anti active JNK polyclonal antibody purified via affinity chromatography (Protein A) from rabbit serum, conc.: 0.02 mg/ml, equiv. 130 nM. Storage buffer: 10 mM Hepes pH 7.5, 150 mM NaCl, 100 µg/ml BSA, 50 % glycerol, store at -70 °C. The stock should be aliquoted in 10 µl aliquots and kept at -20°C. Working solution: 1:20 dilution in 1x Assay-Buffer/0.1% Pluronic

### Reagents and buffers:

*Assay buffer:*
*10x HEPES-Assay-Buffer pH 7.5*
*500mM HEPES*
*1mM EGTA*
*100mM DTT*
*62.5mM NaCl*

Dissolve 770mg DTT (FLUKA cat. no. 43815; MW 154.25g/mol) [final concentration 100mM] in 30 ml of Millipore water, add 625µl of 5M NaCl₂ [final concentration 62.5mM], 19.02mg EGTA (Merck cat. No.1.06404; MW 380.35g/mol) [final concentration 1mM] and 5.96g HEPES (FLUKA cat. no. 54457) [final concentration 500mM] and adjust pH with 1M NaOH to pH 7.5. Top up to 50 ml with Millipore water. The buffer will be filtered sterile (0.22µm) aliquoted to 1.5ml and should be stored at - 20°C.

### 1M MgCl₂:

Dissolve 10.2 g MgCl₂.6H₂O (FLUKA cat. no. 63068) in 50 ml of Millipore water. Buffer should be filtered (0.42µm) and can be stored at 4°C for several months.

### 1% (w/v) Pluronic F-127

Dissolve 0.5 g Pluronic (Sigma, cat. no. P-2443) in 50 ml of Millipore water. Stir gently to get a clear solution. Solution should be filtered (0.42µm) and can be stored at 4°C for several months.

### 1x HEPES-Assay-Buffer/0.1% Pluronic pH 7.5: (15ml):

*50mM HEPES*
*0.1mM EGTA*
*10mM DTT*
*0.1% Pluronic*

Add 1.5 ml 1% (w/v) Pluronic F-127 in water and 1.5 ml of 10x HEPES-Assay-Buffer pH 7.5.to 12 ml of Millipore water. Stir the mixture and check the pH value. The mixture could be stored at 4°C for 1-2 weeks.

### 0.5M EDTA (50ml) :

Dissolve 9.309g EDTA (Roche Molecular Biochemicals, cat. no. 808288) in 40 ml of Millipore water and adjust pH to 8-9 with 10M NaOH in order to get a clear solution. Top up to 50 ml with Millipore water. Buffer should be filtered (0.42µm) and can be stored at 4°C for several months.

### 10mM ATP (15ml) :

Dissolve 9.1mg ATP (Roche Molecular Biochemicals, cat. no. 126888) in 1.5 ml 1x HEPES-Assay-Buffer/0.1% Pluronic pH 7.5. The solution should be filtered (0.42µm) and can be stored at - 20°C for 2 weeks.

### DMSO: 100% DMSO

The solvent was purchased from SIGMA (cat. No. P-2650), sterile filtered.

## Claims

1. A process for detecting threonine or serine kinase activity in an immunoassay comprising the following steps:
a) providing a protein or peptide comprising the sequence motif
-Z-X-Y- or -Y-X-Z-
wherein
Z = threonine or serine
X = a sequence of between 1 and 1000 amino acids which may be the same or different
Y = tyrosine, threonine or serine
as a substrate for threonine or serine kinase, said protein or peptide being pre-phosphorylated at the Y position;
b) incubating the protein or peptide with a phosphate donor and a threonine or serine kinase to form a bis-phosphorylated protein or peptide;
c) adding an antibody having a specificity to the peptide or protein that has been phosphorylated at threonine or serine in the Z position; and
d) detecting the threonine or serine kinase activity.

2. The process according to claim 1, wherein the phosphate donor is ATP, GTP, or a synthetic cosubstrate.

3. The process according to claim 1 or 2, wherein the immunoassay is performed as a direct binding immunoassay, preferably a homogeneous direct binding immunoassay.

4. The process according to claim 3, wherein a labelled peptide or protein is used.

5. The process according to claim 3, wherein a labelled antibody is used.

6. The process according to claim 4 or 5, wherein the peptide/protein or antibody is labelled by a luminescent tag, a radioactive marker, a reporter enzyme or an affinity ligand.

7. The process according to claim 1 or 2, wherein the immunoassay is performed as an indirect binding immunoassay, preferably a homogeneous indirect binding immunoassay.

8. The process according to claim 7, wherein a labelled bis-phosphorylated ligand is added to compete with the bis-phosphorylated protein or peptide for binding to the antibody.

9. The process according to claim 8, wherein the bis-phosphorylated ligand is labelled by a luminescent tag, a radioactive marker, a reporter enzyme or an affinity ligand.

10. The process according to claim 9 wherein the ligand is 5-Tamra-Ahx-Lys-Phe-Met-Met-pThr-Pro-pTyr-Val-Val-Thr-Arg-NH₂.

11. The process according to at least one of claims 1 to 10, wherein the assay is performed as a fluorescence immunoassay, in particular a fluorescence polarization immunoassay, a fluorescence correlation spectroscopic assay, or a fluorescence intensity distribution assay.

12. The process according to at least one of claims 1 to 11, wherein X in the sequence motif comprises proline or glutamate or glycine.

13. The process according to at least one of claims 1 to 12, wherein the protein provided is JNK1, JNK2 or JNK3 protein.

14. The process according to at least one of claims 1 to 12, wherein the peptide provided includes sequences identical to those of the JNK1, JNK2 or JNK3 active-site loop.

15. The process according to claims 1 to 12 wherein the peptide comprises the amino acid sequence H-Lys-Phe-Met-Met-Thr-Pro-pTyr-Val-Val-Thr-Arg-NH₂ (p means phosphorylated).

16. The process according to claim 1 to 12 wherein the peptide is composed of the amino acid sequence H-Lys-Phe-Met-Met-Thr-Pro-pTyr-Val-Val-Thr-Arg-NH₂ (p means phosphorylated).

17. The process according to at least one of claims 1 to 16, wherein the incubation of the protein or peptide is carried out in the presence of a threonine kinase.

18. The process according to claim 17 wherein the threonine kinase is a mitogen-activated protein kinase kinase (MKK)(also named stress activated protein kinase kinase, SKK).

19. The process according to claim 18 wherein the kinase is MKK7 (also named SKK4).

20. The process according to at least one of claims 1 to 19, wherein the antibody is a monoclonal or polyclonal antibody.

21. The process according to claim 20 wherein the antibody is a polyclonal antibody.

22. The process according to claim 21 wherein the antibody is a polyclonal antibody specific for active JNK.

23. A kit for detecting threonine or serine kinase activity in an immunoassay comprising the following components:
- a threonine or serine kinase;
- a substrate as defined in claim 1;
- an antibody as defined in claim 1; and
- reaction buffers including a phosphate donor, preferably ATP.

24. A kit according to claim 23 further comprising a luminescently labelled ligand, said ligand comprising the following sequence motif
-Z-X-Y- or -Y-X-Z-
wherein
Z = threonine or serine
X = a sequence of between 1 and 1000 amino acids which may be the same or different
Y = tyrosine, threonine or serine
said protein or peptide ligand being pre-phosphorylated at the Z and Y positions;

25. A use of the assay process according to at least one of the claims 1 to 22 or the kit according to claims 23 to 24 for screening modulators for threonine or serine kinase activity, in particular inhibitors for a threonine or serine kinase.

26. A luminescent labelled ligand for a serine/threonine kinase comprising the sequence motif according to claim 24.
